# EUROPEAN PATENT SPECIFICATION

(11) **EP 2 222 789 B1**
(45) Date of publication and mention of the grant of the patent: **13.07.2016**
(21) Application number: 08857046.0
(22) Date of filing: 25.11.2008
(51) Int. Cl.: C08L 83/04, C08L 83/00, C08K 3/36, C08J 5/00, C08J 5/02, C08L 83/08, A61Q 15/00, A61K 8/898, A61K 8/02, A61K 8/25, A61K 8/891

(54) **SILICONE POLYMER-SILICA SYSTEM**
SILIKONPOLYMER-SILICIUMDIOXID-SYSTEM
SYSTÈME DE POLYMÈRE DE SILICONE-SILICE

(30) Priority: 30.11.2007 US 991556 P; 21.11.2008 US 275940
(43) Date of publication of application: 01.09.2010
(73) Proprietor: Coty S.A.S., 75008 Paris (FR)
(72) Inventor: BARONE, Salvatore, J., Staten Island, NY 10309 (US); MATEU, Juan, R., Oak Ridge, NJ 07438 (US); MACCHIO, Ralph, Sparta, NJ 07871 (US); STAINA, Irina, Branchburg, NJ 08876 (US)
(74) Representative: Curley, Donnacha John
(86) International application number: PCT/US2008/013126
(87) International publication number: WO 2009/073122

(56) References cited:
- WO-A1-02/069923
- WO-A1-2005/079741
- JP-A- 2002 003 337
- US-A- 4 806 338
- US-A- 5 194 262
- US-A- 5 830 447
- US-A- 5 830 447
- US-A- 5 916 548
- US-A- 5 965 113
- US-A- 5 968 489
- US-A- 5 976 514
- US-A1- 2003 202 949
- US-A1- 2007 148 116
- US-B1- 6 251 413
- AL DISAPIO ET AL: "Silicones: use of substantive properties on skin and hair", INTERNATIONAL JOURNAL OF COSMETIC SCIENCE, vol. 10, no. 2, 1 April 1988 (1988-04-01), pages 75-89, XP055110799, ISSN: 0142-5463, DOI: 10.1111/j.1467-2494.1988.tb00004.x
- DOW CORNING ET AL: 'Product Information Personal Care 9040 Silicone Elastomer Blend', [Online] 26 September 2014, XP055147388 Retrieved from the Internet: <URL:http://www.dowcorning.com/DataFiles/09 0276fe801bf9c7.pdf> [retrieved on 2014-10-17]

## Description

### Field of the Invention

Embodiments of the invention described herein relate to an antiperspirant solid stick with a wax matrix, the antiperspirant solid stick having a chemical system comprising amodimethicone, dimethicone gum and silica.

### Background

Antiperspirants have, as a principle function, a requirement to eliminate adverse effects of perspiration. Antiperspirant function has frequently been associated with undesirable side effects.

For instance US 2003/0202949 discloses an antiperspirant solid stick comprising dimethicone gum and silica.

### Summary

Embodiments of the invention further comprise an antiperspirant comprising a chemical system comprising amodimethicone; dimethicone gum; and silica. where the antiperspirant is a solid stick. In one embodiment, the antiperspirant is a deodorant antiperspirant.

Antiperspirants formulated from embodiments of the chemical system described herein are expected to have increased efficacy and enhanced residual skin feel due to the presence of amodimethicone, improved skin adherence due to the presence of dimethicone gum and an improved overall structure due to the presence of silica. Use of silica further improves the efficacy of solid stick antiperspirants.

### Description

Invention embodiments are described in sufficient detail to enable those skilled in the art to practice the invention. Other embodiments may be utilized and structural, logical, chemical, and other changes may be made without departing from the scope of the invention discussed herein. The following detailed description is, therefore, not to be taken in a limiting sense, and the scope of the invention discussed herein is defined only by the appended claims.

Embodiments of the invention described herein include a base system for use in antiperspirant solid stick compositions,

The base system includes a blend of amodimethicone, dimethicone gum and silica. The concentration of each component in the system is variable and depends upon the application of the base system. A specific concentration may be used in order to maximize efficacy, while minimizing cost and stability issues.

Amodimethicone or aminopropyl dimethicone is an amine-containing silicone polymer useful for multi-product applications. See, for example, U.S. Patent No. 6,649,151 to Applicant, which issued on November 18, 2003. In the acidic antiperspirant environment, the amine groups in amodimethicone are positively charged. The positive charge gives the dimethicone additional properties not usually associated with silicone polymers. These properties are useful across all the antiperspirant product forms. For example, amodimethicone is a potent water absorber and further acts as an emulsifier to stabilize oil (silicone) and water mixtures. The ability to act as an emulsifier provides a pathway for water to penetrate the oil phase and decrease the "water-proofing" affect associated with silicone and water insoluble oils. As a result, efficacy is increased, because distribution of the antiperspirant active is improved, thus making it more available to diffuse into and block the sweat ducts. The positive charge also makes the silicone more substantive to skin for enhanced residual skin feel.

Dimethicone gum or dimethicone is a high molecular weight silicone polymer. Dimethicone gum is a film former useful in all forms of antiperspirants which promotes better adherence of antiperspirant actives to the skin and reduces rub off. In sprays, the high molecular weight of the polymer alters the physical properties of aerosolized droplets resulting in improved adherence to the skin, making them less likely to bounce off the skin. As a result, more of the product sticks to the skin and does not bounce off. An equal amount of the product containing the dimethicone gum dispensed from an aerosol container will deposit more antiperspirant active on the skin than a product not containing the dimethicone gum. Generally, more antiperspirant active means better wetness control.

Silica (SiO₂) is an inorganic oxide of silicon. Silica promotes better structures for complex mixtures. Silica is useful across all antiperspirant product forms in the moisture absorbing complex as a binder to help hold the components together. In this way, the complex maintains its uniform properties upon storage and in the final product. Silica further modifies and improves the wax matrix which provides the structure of an antiperspirant stick. The improved matrix allows additional moisture absorbing complex to be added to the stick, thus improving efficacy of the stick.

The above components, namely amodimethicone, dimethicone gum and silica, may be combined in any suitable concentration depending on the particular application. In one embodiment, the amodimethicone is present in an amount ranging from 1 to 99% by weight, including any specific amount, such as 1.1, 1.2, 1.3, 1.4% by weight, and so forth, up to 99.0% by weight. In one embodiment, dimethicone gum is present in an amount ranging from 1 to 99% by weight, including any specific amount, such as 1.1, 1.2, 1.3, 1.4 % by weight, and so forth, up to 99.0% by weight. In one embodiment, silica is present in an amount ranging from 1 to 99% by weight, including any specific amount, such as 1.1, 1.2, 1.3, 1.4 % by weight, and so forth, up to 99.0% by weight.

Additional components useful in antiperspirants may also be combined with the above components as is known in the art. Such additional components include, but are not limited to, components useful for providing uniform application and resistance to staining. It is desirable that antiperspirants also be gentle to skin and minimize loss of moisture from skin to which antiperspirant is applied. In one embodiment, the additional components may include skin conditioners. In one embodiment, the antiperspirant is an antiperspirant deodorant which contains active components known in the art to contribute to deodorancy. In one embodiment, fragrances known in the art are used. In one embodiment, water, such as de-ionized water, is an optional component in the systems described herein.

It is manifestly intended that embodiments of this invention be limited only by the claims.

## Claims

1. An antiperspirant solid stick with a wax matrix, the antiperspirant solid stick having a chemical system comprising amodimethicone, dimethicone gum and silica.

2. The antiperspirant solid stick of claim 1, wherein the amodimethicone has a concentration of 1 to 99% by weight of the chemical system.

3. The antiperspirant solid stick of claim 1, wherein the dimethicone gum has a concentration of 1 to 99% by weight of the chemical system.

4. The antiperspirant solid stick of claim 1, wherein the silica has a concentration of 1 to 99% by weight of the chemical system.

5. The antiperspirant solid stick of claim 1, wherein the chemical system further comprises water.

6. The antiperspirant solid stick of claim 1, wherein the chemical system further comprises one or more active deodorancy components.

7. The antiperspirant solid stick of any preceding claim, wherein the antiperspirant is a deodorant antiperspirant.

## Patentansprüche

1. Antitranspirant-Feststoff-Stift mit einer Wachsmatrix, wobei der Antitranspirant-Feststoff-Stift ein chemisches System hat, aufweisend Amodimethicone, Dimethicone-Gummi und Silica.

2. Antitranspirant-Feststoff-Stift nach Anspruch 1, wobei das Amodimethicone eine Konzentration von 1 bis 99 Gew.-% des chemischen Systems hat.

3. Antitranspirant-Feststoff-Stift nach Anspruch 1, wobei das Dimethicone-Gummi eine Konzentration von 1 bis 99 Gew.-% des chemischen Systems hat.

4. Antitranspirant-Feststoff-Stift nach Anspruch 1, wobei das Silica eine Konzentration von 1 bis 99 Gew.-% des chemischen Systems hat.

5. Antitranspirant-Feststoff-Stift nach Anspruch 1, wobei das chemische System ferner Wasser aufweist.

6. Antitranspirant-Feststoff-Stift nach Anspruch 1, wobei das chemische System ferner einen oder mehrere aktive deodorierende Bestandteile aufweist.

7. Antitranspirant-Feststoff-Stift nach einem vorangehenden Anspruch, wobei das Antitranspirants ein Deodorant-Antitranspirants ist.

## Revendications

1. Stick solide anti-transpirant ayant une matrice de cire, le stick solide anti-transpirant ayant un système chimique comprenant de l'amodiméthicone, de la gomme de diméthicone et de la silice.

2. Stick solide anti-transpirant selon la revendication 1, dans lequel l'amodiméthicone a une concentration de 1 à 99 % en poids dans le système chimique.

3. Stick solide anti-transpirant selon la revendication 1, dans lequel la gomme de diméthicone a une concentration de 1 à 99 % en poids dans le système chimique.

4. Stick solide anti-transpirant selon la revendication 1, dans lequel la silice a une concentration de 1 à 99 % en poids dans le système chimique.

5. Stick solide anti-transpirant selon la revendication 1, dans lequel le système chimique comprend en outre de l'eau.

6. Stick solide anti-transpirant selon la revendication 1, dans lequel le système chimique comprend en outre un ou plusieurs composants déodorants actifs.

7. Stick solide anti-transpirant selon l'une quelconque des revendications précédentes, dans lequel l'anti-transpirant est un anti-transpirant déodorant.
